# EUROPEAN PATENT APPLICATION

(11) **EP 1 788 370 A1**
(43) Date of publication of application: **23.05.2007**
(21) Application number: 06014228.8
(22) Date of filing: 10.07.2006
(51) Int. Cl.: G01K 13/00, G01K 1/02, G01K 7/42

(54) **Medical electronic thermometer with progress indicator**

(30) Priority: 22.11.2005 US 284765
(71) Applicant: Covidien AG, 8212 Neuhausen am Rheinfall (CH)
(72) Inventor: Bisch, Michael E., Kirkwood, Missouri 63122 (US); Gierer, Joseph T., Glen Carbon, Illinois 62034 (US)
(74) Representative: Tomlinson, Edward James

(57) **Abstract**

Electronic thermometer for medical use, with a progress indicator (23) is provided. A control circuit (13) of the thermometer performs at least one temperature calculation as a function of a detected temperature and generates a temperature signal representative of the temperature of a subject based on the temperature calculation. Said temperature calculation comprises a temperature prediction algorithm. A progress indicator (23) visually indicates progress of the control circuit in performing the temperature calculation.

## Description

### BACKGROUND

The healthcare field widely uses electronic thermometers for measuring a patient's body temperature. A typical electronic thermometer has a thermistor or other temperature sensitive element contained within an elongated shaft portion of a probe. In one version, the probe includes a cup-shaped aluminum tip at its free end. A thermistor is placed in thermal contact with the aluminum tip inside the probe. When the free end of the probe is placed in, for example, a patient's mouth (or rectum or axilla), the tip heats up and the thermistor measures the temperature of the tip to obtain a measurement of the patient's body temperature. Additional electronics connected to these electronic sensor components may be contained within a base unit connected by wire to the shaft portion or may be contained within a handle of the shaft portion. Electronic components receive input from the sensor components to compute the patient's temperature. The thermometer typically displays the patient's temperature on a visual output device, such as a seven segment numerical display device. Additional features of known electronic thermometers include an audible temperature level notification (e.g., a beep or tone alert signal). A disposable cover or sheath is often fitted over the shaft portion and disposed of after each use of the thermometer for sanitary reasons.

Electronic thermometers have many advantages over conventional thermometers and have essentially replaced the use of conventional glass thermometers in the healthcare field. One advantage of electronic thermometers over their conventional glass counterparts is the speed at which a temperature reading can be taken. Several procedures are used to promote a rapid measurement of the subject temperature. One technique employed is to use predictive algorithms as part of thermometer logic to extrapolate the temperature measurement from the thermistor in contact with the tip, to arrive at a temperature reading in advance of the tip reaching equilibrium with the body temperature. Another technique that can be employed simultaneously with a predictive algorithm is to heat the probe to near the body temperature, so that the portion of the probe away from the tip does not act as a heat sink. This allows the tip to reach a temperature close to the body temperature more rapidly. Heating with the probe can be accomplished by a resistor placed in contact with the probe. Another thermistor may be placed in contact with the probe to measure the amount of heat provided by the resistor for controlling the heating. It is also known to use an isolator to reduce heat loss from the tip to other parts of the probe. For example, commonly assigned U.S. Patent No. 6,839,651, the entire disclosure of which is incorporated herein by reference, discloses a prediction type electronic thermometer having an actively controlled heater element thermally isolating the probe tip from the probe shaft.

Although most predictive thermometers provide an activity indication during a prediction measurement to indicate that measurement and prediction activity are occurring, there is no way for a user to know how far along the process has progressed. Further, the thermometer can experience interruptions in the data/temperature trend that cause the algorithm to restart, and for which there is no indication. For example, if the probe is moved within the patient's mouth to a region having a different temperature (e.g., from under the tongue to not under it), the algorithm must restart. Conventional electronic thermometers provide no way for the user to know when anything has interrupted the prediction process.

### SUMMARY

Embodiments of the invention overcome one or more deficiencies in known systems by providing a progress indicator that will visually represent, in real time, how close a device is to producing a correct reading. In the case where the invention is embodied as an electronic thermometer the invention provides a progress indicator that will visually represent how close the thermometer is to producing a temperature reading. For predictive thermometers, aspects of the invention provide an indication of how close the prediction is to completion. In addition, one embodiment of the invention provides visual feedback if the thermometer must restart or reset for some reason. Moreover, the features of the present invention described herein are user friendly and intuitive as well as being economically feasible and commercially practical.

Briefly described, an electronic thermometer embodying aspects of the invention has a probe adapted to be heated by a subject for use in measuring the temperature of the subject and at least one temperature sensor for detecting the temperature of the probe during operation. A control circuit responsive to the temperature sensor performs at least one temperature calculation as a function of the detected temperature of the probe and generates a temperature signal representative of the temperature of the subject based on the temperature calculation. In addition, the thermometer includes a progress indicator for visually indicating progress of the control circuit in performing the temperature calculation.

According to another aspect of the invention, a method of indicating status of an electronic thermometer includes performing at least one temperature calculation as a function of a detected temperature of a probe, which is adapted to be heated by a subject for use in measuring the temperature of the subject. The method also includes defining a plurality of states, each of which corresponds to an amount of completion of the temperature calculation. The method further includes visually indicating progress of the temperature calculation based on the defined states.

Yet another aspect of the invention is directed to a medical device that has a control circuit configured to perform at least one operation over time and a progress indicator for visually indicating progress of the control circuit in performing the operation.

Other features will be in part apparent and in part pointed out hereinafter.

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

### BRIEF DESCRIPTION OF THE DRAWINGS

FIG. 1 is a perspective of an electronic thermometer according to embodiments of the invention.

FIG. 2 is an exemplary progress indicator according to an embodiment of the invention.

FIG. 3 is an exemplary progress indicator according to another embodiment of the invention.

FIG. 4 is an exemplary flow diagram illustrating a progress determination of a temperature prediction process according to an embodiment of the invention.

FIG. 5 and FIG. 6 are exemplary flow diagrams illustrating a stability variance determination of a temperature prediction process according to another embodiment of the invention.

Corresponding reference characters indicate corresponding parts throughout the drawings.

### DETAILED DESCRIPTION

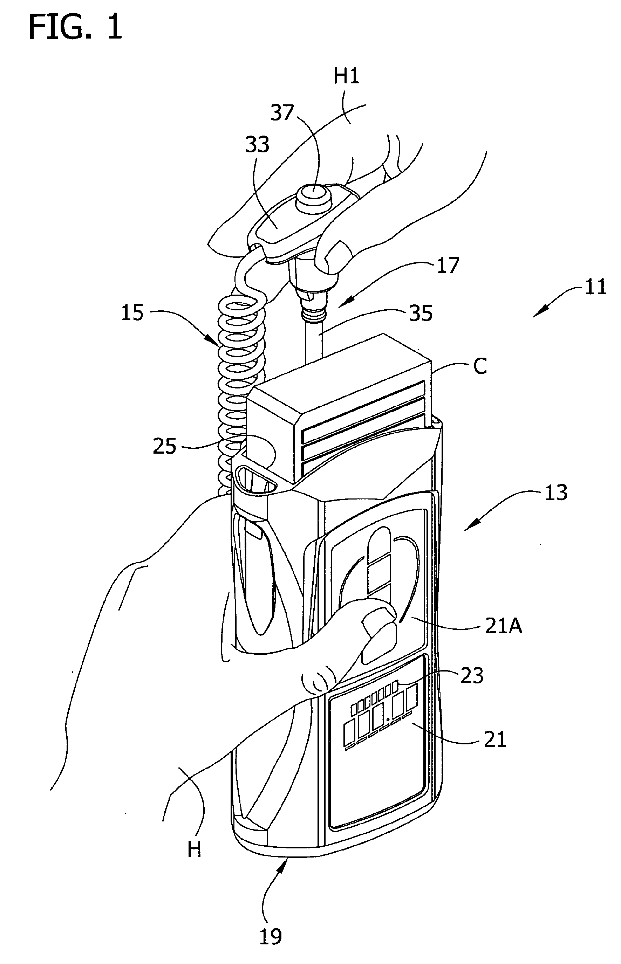

Referring now to the drawings and in particular to FIG. 1, an electronic thermometer constructed according to the principles of the present invention is indicated generally at 11. The electronic thermometer comprises a temperature control circuit or unit, indicated generally at 13, that is sized and shaped to be held comfortably in the hand H. The control unit 13 (broadly, "a base unit") is connected by a helical cord 15 to a probe 17 (the reference numerals indicating their subjects generally). The probe 17 is constructed for contacting the subject (e.g., a patient) and sending signals to the control unit 13 representative of the temperature. The control unit 13 receives the signals from probe 17 and uses them to calculate the temperature. Suitable circuitry for performing these calculations is contained within a housing 19 of the control unit 13. The logic in the circuitry may include a predictive algorithm for rapidly ascertaining a final temperature of the patient. The circuitry makes the calculated temperature appear on a LCD display 21 on the front of the housing 19. Other information desirably can appear on the display 21, as will be appreciated by those of ordinary skill in the art. A panel 21 A of buttons for operating the thermometer 11 is located just above the display 21. In the embodiment of FIG. 1, display 21 includes a progress indicator 23.

Those skilled in the art are familiar with various user interfaces for displaying information to a user and receiving user input. As an example, display 21 may be a graphical user interface having a touch screen by which the user can provide input. In this example, the panel 21 A could be embodied on display 21 itself.

The housing 19 includes a compartment (not shown) generally at the rear of the housing that can receive a distal portion of the probe 17 into the housing for holding the probe and isolating the distal portion from the environment when not in use. FIG. 1 illustrates probe 17 being pulled by the other hand H1 from the compartment in preparation for use. The housing 19 also has a receptacle 25 that receives a suitable container such as a carton C of probe covers (not shown). In use, the top of the carton C is removed, exposing open ends of the probe covers. The distal portion of probe 17 can be inserted into the open end of the carton C and one of the probe covers can be captured (e.g., snapped into) an annular recess. The probe 17 may be protected from contamination by the cover when a user inserts the distal portion of a probe shaft 35 into, for example, a patient's mouth. When depressed, a button 37 on the probe handle 33 causes pushers located at the junction of the probe shaft 35 and a handle 33 of probe 17 to move for releasing the probe cover from probe shaft 35. Subsequent to use, the probe cover is discarded. Other ways of capturing and releasing probe covers may be used without departing from the scope of the present invention.

An aluminum tip at the distal end of probe shaft 35 is heated up by the patient and the temperature of the tip is detected, as will be described more fully hereinafter. The probe cover is preferably made of highly thermally conductive material, at least at the portion covering the tip, so that the tip can be rapidly heated by the patient. Batteries (not shown) may be used to power a tip thermistor (not shown), separator thermistor (not shown), and/or resistor (not shown) preferably located in the housing 19 of thermometer 11. It will be understood that other suitable power sources could be employed. The power source need not be located in the control unit housing 19. In general, the tip thermistor generates a signal that is representative of the temperature of the tip. The signal is transmitted by one or more electrical conductors to the circuitry in housing 19. As described above, the resistor is powered by the batteries and heats a separator (not shown) so that the aluminum tip can reach the temperature of the patient more rapidly. Monitoring the temperature of the separator with the separator thermistor allows the heating of the resistor to be controlled to achieve optimum results. For instance, the separator can be initially rapidly heated, but then heated intermittently as the separator nears or reaches a preselected temperature. The function and operation of these components are known to those of ordinary skill in the art. It will be appreciated that various electrical components (not shown) and other arrangements and numbers of components may be used without departing from the scope of the present invention.

For example, commonly assigned U.S. Patent No. 6,634,789, U.S. Patent No. 6,839,651, and U.S. Patent Application No. 11/266,548, and U.S. Patent Application No. 11/265,984, the entire disclosures of which are incorporated herein by reference, disclose electronic thermometers.

The response time of electronic thermometers has also been improved by methods that do not involve heating the probe shaft or tip. Predictive type thermometers are known, for example, wherein a set of early temperature measurements are read by the electronics of the thermometer and a mathematical algorithm is applied to extrapolate to a final estimated equilibrium temperature. Various prediction type thermometers are known that improve response time and provide accurate temperature estimations. Still other methods of improving the response time of electronic thermometers are known which combine heating methods with prediction methods. Using predictive techniques, the patient's temperature reading is taken in a significantly shorter time period, for example thirty seconds, compared to several minutes required for conventional Mercury thermometers.

Predictive thermometers use a numerical algorithm to accelerate the speed with which a patient's body temperature is acquired. The algorithm uses the temperature history of the thermometer's probe and projects where the final temperature reading will be. In order for the software to be satisfied that an accurate temperature has been predicted, a certain number of final temperature projections must fall within a certain range or tolerance of each other.

Despite the response time improvements over glass thermometers, typical electronic thermometers can still have unacceptably long response times. Delay in providing a temperature reading often results from the patient inadvertently repositioning probe 17 during the measurement. For example, if the patient moves the probe to a region having a different temperature (e.g., from under the tongue to not under it) the algorithm may need to restart. This may also be the case if the patient draws in a breath of air over the probe. As described above, conventional predictive thermometers at most provide an activity indication during a prediction measurement to indicate that measurement and prediction activity are occurring. But there is no way for a user of a conventional thermometer to know how far along the process has progressed. Further, if the thermometer experiences interruptions in the data/temperature trend that cause the algorithm to restart, there is no indication by a conventional thermometer. Thus, users desire progress information.

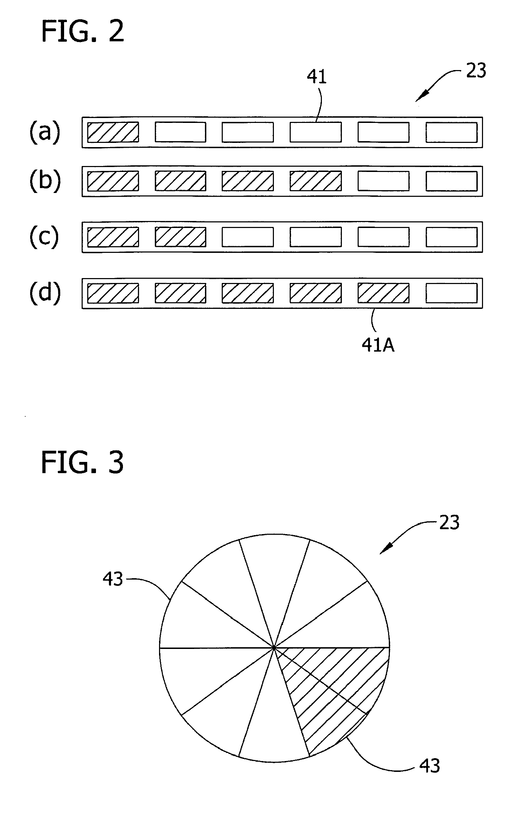

According to aspects of the invention, the progress indicator 23 provides a user friendly, visual indication of relative completion of the prediction. As shown in the examples of FIGS. 2 and 3, progress indicator 23 may take the form of any type of visual indication or display, such as a progress bar (oriented horizontally or vertically on the display screen 21) (see FIG. 2) or a pie graph (see FIG. 3). For example, indicator 23 may be a series of icons or segments 41 forming a bar, a segmented single bar, a continuous bar that is progressively filled in or illuminated, or a continuous bar of variable length, all within the scope of the present invention. In these exemplary embodiments, indicator 23 essentially takes the form of a variable length bar that provides a visual characteristic for indicating a relative amount of completion of the temperature calculations. In an alternative embodiment, indicator 23 may be a pie graph or "pin wheel" having pieces 43 that are progressively filled in piece-by-piece as thermometer 11 performs the temperature calculations. Similarly to a bar-type indicator, the pie graph may also be filled in progressively in a continuous manner. Those skilled in the art are familiar with generating graphical user interface components for displaying symbols such as those contemplated herein on an LCD screen or other type of display in color, gray scale, or monochrome.

In one embodiment, display 21 displays the temperature as estimated during the operation of progress indicator 23. This temperature may be updated as progress continues. In the alternative, thermometer 11 waits until completion of the temperature calculations to display the temperature reading.

Referring now to the exemplary embodiment of FIG. 2, indicator 23, in the form of a variable length or segmented bar, "marches" along as the sequence of ongoing predictions start to converge to an answer (i.e., the trend flattens out and becomes more consistent). As shown in FIG. 2(a), progress indicator 23 has a single segment 41 filled in, which indicates that the temperature calculations are just beginning. It is to be understood that each segment 41 may be embodied by an LED, a shaded or colored icon or portion of the bar, a bright or illuminated icon or portion of the bar, a blinking or solid icon or portion of the bar, or the like to visually distinguish itself from the remainder of the bar.
Moreover, segments 41 may abut each other or be separated and may be grouped or boxed to indicate the full length of the bar. FIG. 2(b) illustrate that thermometer 11 has completed about half of the necessary calculations for rendering a final temperature. FIG. 2(c) illustrates that an interruption to the temperature calculations may have occurred, resulting in a fall back in progress. In other words, if the trend is interrupted for any reason and the prediction must re-start, progress indicator 23 provides such an indication by becoming less filled in and resets as appropriate. Conversely, an immediate full bar may be used to indicate an error (e.g., bad placement). At FIG. 2(d), only the last segment 41 remains to indicate the thermometer 11 is nearing completion of its temperature calculations. In this embodiment, segment 41 A may be blinking or otherwise visually distinguishable from the other filled in segments 41 to indicate the current relative status of completion.

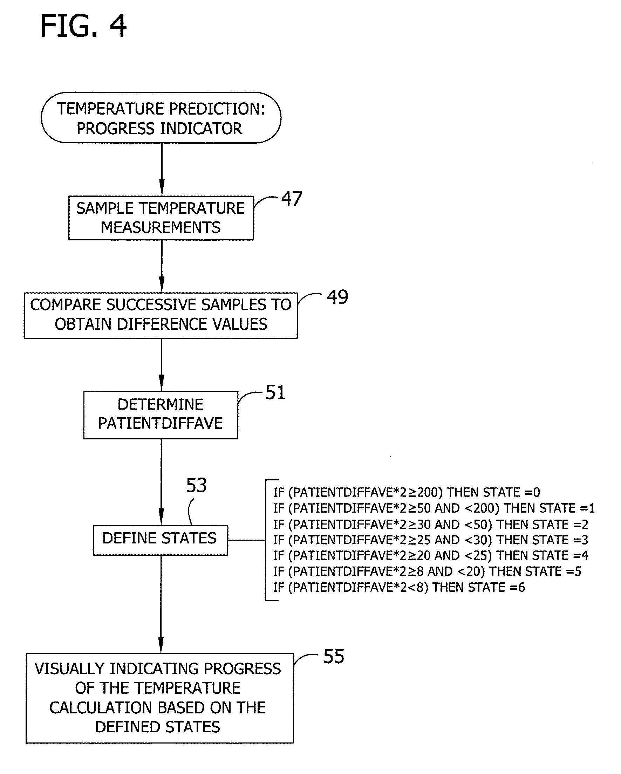

As shown in the exemplary flow diagram of FIG. 4, in one embodiment of the invention, control unit 13 continuously samples the temperature thermistor at 47 during a prediction determination and generates progress indicator 23 as a function of the patient thermistor. Using a software loop, control unit 13 saves and compares the last several samples at 49 for determining the trend toward a final temperature measurement. In other words, variables can be used to see if consecutive thermistor readings are getting closer together. For example, control unit 13 uses the following at 51:
PatientCountLast = PatientCountNow
PatientCountNow = ThermistorValueNow
PatientDiff3 = PatientDiff2
PatientDiff2 = PatientDiff1
PatientDiff1 = PatientCountNow-PatientCountLast
PatientDiffAve = (PatientDiff3+PatientDiff2+PatientDiff1)/3

The value PatientDiffAve in this example is the average of the last three sets of differences accumulated.

As described above, progress indicator 23 may be any number of discrete pieces and may take many different shapes or appearances. The bar shown in FIG. 2 provides a suitable implementation of progress indicator 23 because users are familiar with the use of a bar in the context of many computer applications to indicate "busy" or "activity." For this example, seven segments 41 are used to define a complete bar. The various states of completion of progress indicator 23 are numbered 0-6 in this example where the state number represents the number of pieces or segments 41 that are "on" during that state. For example, in state 0 there are no segments turned on; in state 1 there is one segment turned on; and so forth. Thus, a progress bar begins at state 0 and sequentially progresses to state 1, 2, 3, 4, 5, and 6. At state 6, a complete bar indicates that the activity is substantially 100% complete. In one embodiment, the states are defined as a function of temperature, time, etc.

According to aspects of the invention, there may be many ways to determine when and how the states progress from 0 to 6. For example, if an activity is expected to take a relatively consistent amount of time, the states might progress based on a time interval (with the final state being slightly delayed until the activity is complete). In an alternative embodiment, state progression may be defined according to the PatientDiffAve value described above. Referring again to FIG. 4, exemplary states are defined at 53 as follows:
if (PatientDiffAve*2≥ 200) then State =0
if (PatientDiffAve*2≥ 50 and <200) then State =1
if (PatientDiffAve*2≥ 30 and <50) then State =2
if (PatientDiffAve*2≥ 25 and <30) then State =3
if (PatientDiffAve*2≥ 20 and <25) then State =4
if (PatientDiffAve*2≥ 8 and <20) then State =5
if (PatientDiffAve*2 <8) then State =6

Proceeding to the operation illustrated in FIG. 4 at 55, progress indicator 23 displays a visual indication of the progress of the temperature calculation based on the defined state. Different ranges may be selected to cause progress indicator 23 to behave differently as PatientDiffAve changes. Likewise, many combinations or calculations based on PatientDiff1, PatientDiff2, PatientDiff3, PatientDiffAve, PatientCountNow and PatientCountLast may be used. Moreover, one embodiment of progress indicator 23 does not display progress unless certain threshold conditions are met (e.g., sensing that the probe 17 of thermometer 11 has been placed in the patient; the thermistor reading is greater than a certain baseline value; and/or the last several readings have all been increasing).

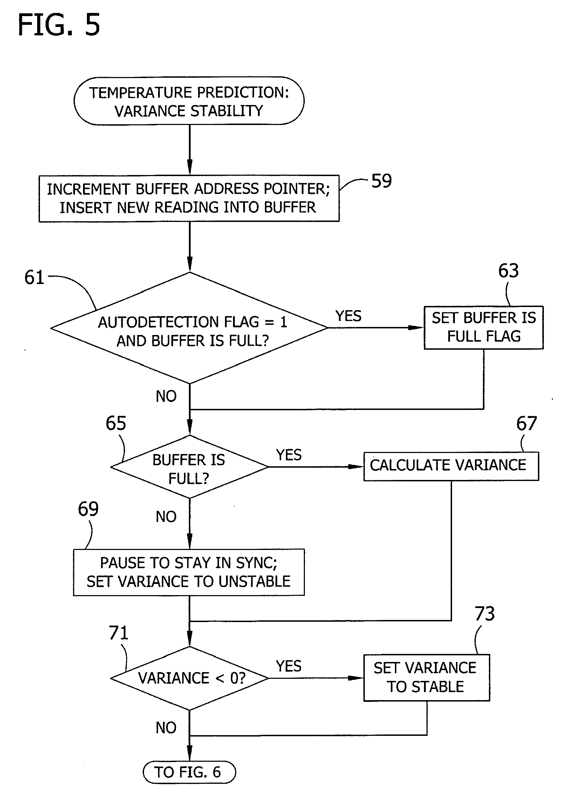

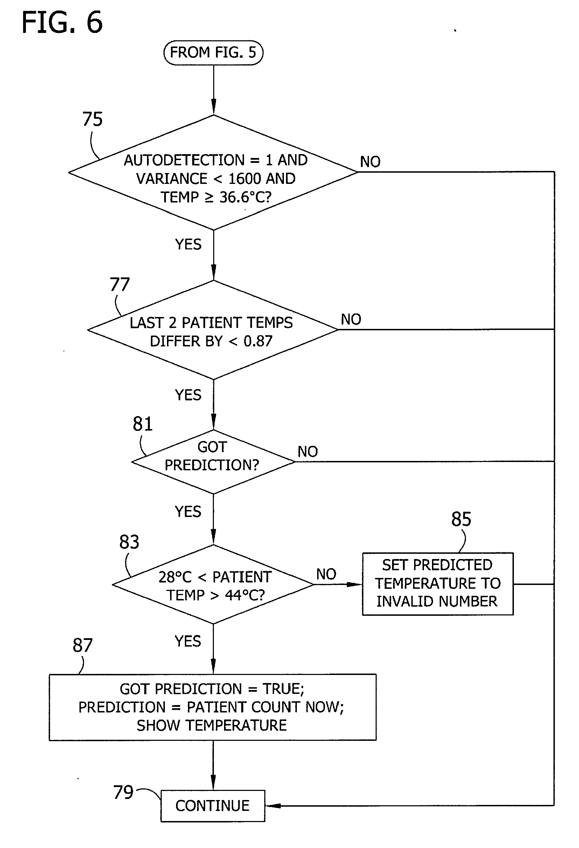

In an alternative embodiment, a different intermediate variable within the prediction algorithm may be used in manner similar to PatientDiffAve. For example, a stability calculation may be continuously made during the prediction algorithm similarly to PatientDiffAve. Replacing PatientDiffAve with this stability factor, or using it in a generally similar manner to determine progress bar state is contemplated by the invention. For reference, an exemplary variance stability prediction flowchart is shown in FIGS. 5 and 6.

Referring now to FIG. 5, control unit 13 computes a moving variance of patient temperatures. If the variance is low enough, it reports the temperature as is (without doing any predictions). In one embodiment, control unit 13 calculates a moving standard deviation to determine if a temperature is stable. However, to save execution time, taking the square root as would normally be done for the standard deviation may be omitted. Instead, the constant that is used for comparison is squared. Hence, the term variance instead of standard deviation is used. Beginning at 59, control unit 13 increment its buffer address pointer and inserts a new reading into the buffer. Proceeding to 61, control unit 13 reads an AutoDetection Flag and checks whether the buffer is full. If so, control unit 13 sets a flag at 63 to indicate that the buffer is full. If the buffer is full, as determined at 65, control unit 13 calculates the variance. If not, operations pause at 69 to remain synchronized and the variance is set to indicate that it is unstable or that the buffer needs more data. Then, if variance is less than zero at 71, the variance is set to stable at 73.

Proceeding to FIG. 6, if the AutoDetection flag is set to one at 75, the variance is less than 1600, and the temperature is greater than or equal to 36.6°C, control unit 13 determines at 77 whether the last two patient thermistor temperature readings are within 0.87, for example, of each other. On the other hand, if each of the three conditions is not met at 75, control unit 13 proceeds to 79. Continuing at 81, if the last two temperature readings differ by less than 0.87, control unit 13 determines at 81 that a temperature prediction has been made. If not, operations proceed to 79 but otherwise continue at 83 to determine if the patient thermistor temperature readings are within an acceptable range (e.g., 28°C to 44°C). If the readings are outside of this range, control unit 13 considers them to be invalid at 85; if the readings are within this range, control unit 13 considers them to be valid predictions.

Although described primarily in the context of a predictive thermometer, aspects of the invention also apply to a direct measurement mode where the predictive algorithm is turned off. In this situation, progress indicator 23 similarly shows relative completion of stabilization of the measured temperature. The direct measurement mode examines, for example, the convergence of actual temperature rather than predicted temperature.

In operation, thermometer 11 indicates its status by performing at least one temperature calculation as a function of a detected temperature of probe. The probe 17 is adapted to be heated by a subject for use in measuring the temperature of the subject. By defining plurality of states, each of the states corresponding to an amount of completion of the temperature calculation, thermometer 11 is able to indicate progress of the temperature calculation. In one embodiment, each of the defined states corresponds to one or more successive operations performed in the temperature calculation. Advantageously, thermometer 11 visually indicates progress by displaying progress indicator 23 to a user. The progress indicator 23 may be a progress bar having a variable length indicative of a percentage of completion of the temperature calculation. Likewise, progress indicator 23 may have a plurality of segments 41 or 43 that correspond to the defined states and that have a visual characteristic representing the amount of completion of the temperature calculation.

It is to be understood that aspects of the present invention may be applied to medical devices generally. In an alternative embodiment of the invention, a medical device, such as an electronic thermometer, pump, blood pressure monitor, or other medical device that performs an operation over time, includes control circuit 13 and progress indicator 23. The control circuit 13 is configured to perform at least one operation over time and the progress indicator 23 visually indicates the progress of control circuit 13 in performing the operation. In this instance, control circuit 13 executes operations similar to those illustrated in FIGS. 4-6 but modified according to the particular function of the medical device. The control circuit 13 defines a plurality of states that each correspond to an amount of completion of the operation. Based on these defined states, progress indicator 23 visually indicates progress of the operation. For example, a progress bar may be used in conjunction with a pump, such as an enteral feeding pump or infusion pump, to indicate the progress of the delivery of a desired volume of fluid. In another example, a progress bar may be used in conjunction with a blood pressure monitor to indicate the progress of the blood pressure measurement.

The order of execution or performance of the methods illustrated and described herein is not essential, unless otherwise specified. That is, it is contemplated by the inventors that elements of the methods may be performed in any order, unless otherwise specified, and that the methods may include more or less elements than those disclosed herein. For example, it is contemplated that executing or performing a particular element before, contemporaneously with, or after another element is within the scope of the invention.

When introducing elements of the present invention or the embodiments thereof, the articles "a," "an," "the," and "said" are intended to mean that there are one or more of the elements. The terms "comprising," "including," and "having" are intended to be inclusive and mean that there may be additional elements other than the listed elements.

In view of the above, it will be seen that the several objects of the invention are achieved and other advantageous results attained.

As various changes could be made in the above constructions and methods without departing from the scope of embodiments of the invention, it is intended that all matter contained in the above description and shown in the accompanying drawings shall be interpreted as illustrative and not in a limiting sense.

## Claims

1. A medical device comprising:
a control circuit (13) configured to perform at least one operation over time; and
a progress indicator (23) for visually indicating progress of the control circuit (13) in performing the operation.

2. The medical device according to claim 1, wherein the operation comprises a temperature calculation, and further comprising a probe (17) adapted to be heated by a subject for use in measuring the temperature of the subject and at least one temperature sensor for detecting the temperature of the probe (17), and wherein the control circuit (13) is responsive to the temperature sensor and configured to perform the temperature calculation as a function of the detected temperature and to generate a temperature signal representative of the temperature of the subject based on the temperature calculation.

3. The medical device according to claim 2, wherein the control circuit (13) comprises a temperature prediction component configured to calculate a predicted temperature of the subject using an optimized temperature prediction algorithm.

4. The medical device according to claim 2 or claim 3, wherein the progress indicator (23) comprises a progress bar having a variable length indicative of a percentage of completion of the control circuit (13) in performing the temperature calculation.

5. The medical device according to any one of claims 2-4, wherein the progress indicator (23) comprises a plurality of segments (41), each of said segments (41) having a visual characteristic for representing a percentage of completion of the control circuit (13) in performing the temperature calculation.

6. The medical device according to any one of the preceding claims, further comprising a screen (21) on which the progress indicator (23) is displayed.

7. The medical device according to any one of the preceding claims, wherein the progress indicator (23) comprises a graphical user interface component displayed on the screen (21).

8. The medical device according to any one of the preceding claims, wherein the progress indicator (23) comprises a visual characteristic representative of a progress condition, an error condition, or a reset condition.

9. A method of indicating a status of a medical device, said method comprising:
performing (47) at least one operation over time;
defining (53) a plurality of states, each of said states corresponding to an amount of completion of the operation;
visually indicating (55) progress of the operation based on the defined states.

10. The method according to claim 9, wherein performing (47) the operation comprises performing (47) a temperature calculation as a function of a detected temperature of a probe (17), said probe (17) being adapted to be heated by a subject for use in measuring the temperature of the subject.

11. The method according to claim 10, wherein performing (47) the temperature calculation comprises executing a temperature prediction component configured to calculate a predicted temperature of the subject using an optimized temperature prediction algorithm.

12. The method according to claim 10 or claim 11, wherein said progress indicator (23) has a plurality of segments (41), each of said segments (41) corresponding to one of the defined states and having a visual characteristic representing the amount of completion of the temperature calculation.

13. The method according to any one of claims 9-12, wherein each of the defined states corresponds to one or more successive operations performed in the temperature calculation.

14. The method according to any one of claims 9-13, wherein defining (53) the states comprises estimating a time interval for completion of the one or more successive operations corresponding to each of the defined states.

15. The method according to any one of claims 10-14, further comprising sampling (47) the detected temperature, comparing (49) successive samples of the detected temperature to obtain difference values, and averaging (51) a predetermined number of the difference values to calculate the average difference values.

16. The method according to claim 15, wherein defining (53) the states includes assigning progressively decreasing ranges of average difference values to the states, and further comprising comparing the calculated average difference values to the defined states for estimating the amount of completion of the temperature calculation.

17. The method according to any one of claims 9-16, wherein visually indicating (55) progress comprises displaying a progress indicator (23) to a user, said progress indicator (23) comprising a progress bar having a variable length indicative of a percentage of completion of the operation.

18. The method according to any one of claims 9-17, wherein visually indicating (55) progress of the operation comprises displaying a graphical user interface component on a screen (21).

19. The method according to any one of claims 9-18, wherein visually indicating (55) progress of the operation comprises displaying a visual characteristic representative of a progress condition, an error condition, or a reset condition.
